(19) Europäisches Patentamt
European Patent Office
Office européen des brevets

(11) Veröffentlichungsnummer: **0 005 519**
**B1**

(12) # EUROPÄISCHE PATENTSCHRIFT

(45) Veröffentlichungstag der Patentschrift: **13.04.83**

(51) Int. Cl.³: **G 01 N 31/22**, G 01 N 33/50 // C07D213/20, C07D213/26, C07D215/10, C07D217/10

(21) Anmeldenummer: **79101446.7**

(22) Anmeldetag: **11.05.79**

(54) **Schnelltest und Verfahren zur Bestimmung von Ammoniak oder unter Bildung von Ammoniak reagierenden Substraten.**

(30) Priorität: **17.05.78 DE 2821469**

(43) Veröffentlichungstag der Anmeldung: **28.11.79 Patentblatt 79/24**

(45) Bekanntmachung des Hinweises auf die Patenterteilung: **13.04.83 Patentblatt 83/15**

(84) Benannte Vertragsstaaten: **AT BE CH DE FR GB IT LU NL SE**

(56) Entgegenhaltungen:
**DE-A-2 626 367**
**DE-A-2 748 857**
**DE-B-1 240 306**
**DE-B-1 245 619**
**DE-B-2 118 455**
**DE-B-2 249 647**
**US-A-2 632 761**
**RESERACH DISCLOSURE, Nr. 161, September 1977, Hampshire, GB, »Element for analysis of liquids« Nr. 16169, Seiten 70—76* Seiten 70, 71; Figur 4***
**JOURNAL FÜR PRAKTISCHE CHEMIE, Band 317, Nr. 5, 1975, Leipzig, DE, D. KLEMM et al.: »Synthese photochromer 2-(2',4'-Dinitrobenzyl)-pyridine« Seiten 761—770**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Rothe, Anselm, Dr., Im Schwanklingen 20, D-6943 Birkenau (DE)**
Erfinder: **Selle, Adolf Karl, Lärchenweg 12, D-6943 Birkenau (DE)**
Erfinder: **Lange, Hans-Rudolf, Röntgenstrasse 14, D-6840 Lampertheim (DE)**
Erfinder: **Rittersdorf, Walter, Dr., Kasseler Strasse 6, D-6800 Mannheim 31 (DE)**
Erfinder: **Werner, Wolfgang, Dr., Meissener Weg 39, D-6800 Mannheim 42 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Schnelltest und Verfahren zur Bestimmung von Ammoniak oder unter Bildung von Ammoniak reagierenden Substraten

Die Bestimmung des Harnstoffs in Körperflüssigkeiten ist von großer Wichtigkeit für die Diagnose und Verlaufskontrolle von Erkrankungen der Niere. Schon seit längerer Zeit stehen dafür eine Reihe von naßchemischen Analysenverfahren zur Verfügung, die auf folgenden Prinzipien beruhen: Bei einer direkten Methode wird Harnstoff mit Diacetylmonoxim umgesetzt und die Absorption der neu entstandenen Verbindung photometrisch gemessen. Andere, moderne Methoden schalten vor die Farbreaktion einen enzymatischen Schritt und zersetzen mittels des Enzyms Urease den Harnstoff zu Ammoniumcarbonat. Das Ammoniak wird anschließend z. B. in der bekannten Nesslerschen Farbreaktion umgesetzt oder nach Berthelot mit Phenol/Hypochlorit in ein gefärbtes Indophenol überführt. Die Extinktion der jeweils entstandenen farbigen Reaktionsprodukte ist schließlich das Maß für die ursprünglich vorhandene Harnstoffmenge.

Diese Verfahren sind an sich von hinreichender Genauigkeit, erfordern jedoch teure Photometer und geschultes Personal mit ausreichender Erfahrung beim exakten Pipettieren und Umgang mit zum Teil wenig haltbaren und ätzenden Reagenzien. Da diese Verfahren nicht für Vollblut angewandt werden können, sind zusätzliche Laboreinrichtungen zur Gewinnung von Serum bzw. Plasma nötig.

Besonders in Notfällen, z. B. bei einem urämischen Koma, ist es für die einzuleitenden therapeutischen Maßnahmen unerläßlich, in kürzester Zeit ein möglichst genaues Analysenergebnis zu erhalten. Deshalb fehlte es nicht an umfangreichen Bemühungen, auch für die Bestimmung von Harnstoff in Körperflüssigkeiten Methoden zu entwickeln, die es ermöglichen, innerhalb kürzester Zeit ein diagnostisch gut verwertbares Analysenergebnis zu bekommen.

Wegen der Einfachheit der Handhabung, der zum Teil kürzeren Reaktionszeit, der Anwendbarkeit am Krankenbett und im Notfall und der Benutzung durch ungeschultes Personal oder den Patienten selbst haben sich daher in neuerer Zeit Schnellteste in Form von Teststreifen durchgesetzt.

Ein Schnelltest zur Bestimmung von Harnstoff in Körperflüssigkeiten wie Blut, Serum und Plasma wird in der DE-AS 1 240 306 beschrieben. Hierbei sind Urease, pH-Indikator, Puffer und Hilfsstoffe zusammen auf ein Papier imprägniert. Bei Auftropfen einer Körperflüssigkeit wird der darin enthaltene neutral reagierende Harnstoff in alkalisch reagierendes Ammoniumcarbonat gespalten. Die pH-Änderung wird durch eine Farbänderung des pH-Indikators angezeigt, der Harnstoffgehalt der Probe kann durch Vergleich mit Vergleichsfarben geschätzt werden. Trotz einiger Vorzüge — wie einfacher Handhabung und kurzer Reaktionszeit — ist dieser Teststreifen wegen seiner nur schwer unterschiedbaren Reaktionsfarben und wegen des deutlichen Einflusses des Säure-Basen-Haushalts der Körperflüssigkeit auf die Reaktionsfarbe nur für orientierende Schätzungen brauchbar.

Das Problem der Abhängigkeit vom Säure-Basen-Haushalt der Körperflüssigkeit wurde mit einem in der DE-AS 1 245 619 beschriebenen Teststreifen gelöst, bei dem ein saugfähiger Papierstreifen nebeneinander mit drei unterschiedlichen Lösungen imprägniert ist. Bei diesem Teststreifen wird die harnstoffhaltige Lösung zunächst in einem ureasehaltigen Testbezirk aufgesaugt und der Harnstoff darin in Ammoniumcarbonat umgesetzt. Durch Kapillarkräfte wird die Lösung dann in einen benachbarten alkalischen Testbezirk überführt, der aus dem Ammoniumcarbonat gasförmiges Ammoniak freisetzt, das über den den Teststreifen umgebenden Gasraum auf einen dritten Testbezirk übertritt und den darauf befindlichen pH-Indikator entsprechend der Konzentration des Harnstoffs verfärbt. Durch eine etwa 2 mm breite hydrophobe Zone zwischen dem zweiten und dritten Testbezirk wird ein Übertreten der alkalischen Lösung verhindert. Da so nur gasförmiger Ammoniak auf das dritte Testfeld übertritt, stören andere Basen oder Säuren nicht. Weiterhin kann durch Pufferung das erste Testfeld auf einen für die Urease optimalen pH-Wert eingestellt werden, solange die Alkalisierung auf dem zweiten Testbezirk ausreicht, um das Ammoniak quantitativ freizusetzen.

Trotz diagnostisch gut verwertbarer Ergebnisse besitzt dieser Teststreifen eine Reihe von Nachteilen. Die Bestimmung kann nur mit Serum oder Plasma, nicht jedoch mit Blut durchgeführt werden, da die Blutkörperchen die Chromatographie stören. Das setzt ein Zentrifugieren des Blutes voraus, d. h. die benötigte Probenmenge ist groß und kann nur vom Arzt oder entsprechend gut geschultem Personal gewonnen werden. Um die Überführung des Ammoniaks durch den Gasraum reproduzierbar zu machen, muß weiterhin der Teststreifen in eine spezielle Reaktionskammer gehängt und relativ zur Probe während der Reaktionszeit genau fixiert sein. Für die Chromatographie in den Testbezirken 1 und 2 und insbesondere für die Diffusion des Ammoniaks durch den Gasraum ist eine Reaktionszeit von 30 Minuten erforderlich, weshalb dieser Teststreifen kaum noch als Schnelltest zu bezeichnen ist.

In der DE-AS 2 249 647 wird ein ähnlich aufgebauter Teststreifen beschrieben, der einige konstruktive Verbesserungen aufweist. Es können damit auch Bestimmungen in Vollblut vorgenommen werden. Auch dieser Teststreifen erfordert jedoch im Normalfall eine Reaktionszeit von 30 Minuten und benötigt eine Reaktionskammer.

Ein weiterer Nachteil der beiden obengenannten Teststreifen liegt darin, daß die Konzentration des Harnstoffs durch eine visuelle Längenmessung der umgeschlagenen Zone des Indikatorpapiers mit seiner schwer zu beurteilenden, undeutlichen Grenzzone erfolgt und nicht durch die visuelle oder präzise Messung der Farbtiefe mit einem Remissionsphotometer vorgenommen werden kann.

In der DE-OS 2 626 367 wird eine Vorrichtung mit mehreren übereinanderliegenden Schichten beschrieben. Zum Nachweis von Harnstoff bestehen diese aus einer urease- und einen alkalischen Puffer enthaltenden Schicht und einer gasförmiges Ammoniak nachweisenden Indikatorschicht. Um zu verhindern, daß von der Ureaseschicht außer dem gasförmigen Ammoniak unerwünschterweise auch Probenflüssigkeit zur Indikatorzone diffundiert, werden die Ureaseschicht und die darunter befindliche Indikatorschicht durch einen hydrophoben, gasdurchlässigen Film getrennt oder die Indikatorschicht als hydrophober Film gestaltet. Die je nach Harnstoffgehalt der Probe unterschiedlich intensiv gefärbte Indikatorschicht wird remissionsphotometrisch durch die Trägerfolie vermessen und so die Konzentration des Harnstoffs bestimmt.

Um zu Reaktionszeiten von etwa 10 Minuten zu gelangen, muß diese Testvorrichtung wegen der relativ hohen Undurchlässigkeit der Trennschicht bei 40–50°C inkubiert werden. Wegen des damit verbundenen apparativen Aufwandes ist der Einsatz dieser Testvorrichtung als Schnelltest nicht möglich.

In RD Nr. 16, 1977, 70–71, sind mehrschichtige Testvorrichtungen mit einer flüssigkeitsdurchlässigen Abstandsschicht (spacer layer) beschrieben, über die zusätzliche Flüssigkeiten aus einem in oder neben dieser Schicht befindlichen Reservoir eingebracht werden können.

Alle bisher bekannten Teststreifen zur Bestimmung von Harnstoff weisen also noch wesentliche Mängel auf. Es bestand daher für die vorliegende Erfindung die Aufgabe, für den Nachweis von Harnstoff in Körperflüssigkeiten einschließlich Vollblut einen Teststreifen zu entwickeln, der:

- einfach in der Handhabung ist und ohne zusätzliche Reaktionskammern auskommt,
- eine Harnstoffbestimmung nicht nur in Serum bzw. Plasma, sondern auch in geringen Mengen Vollblut erlaubt,
- bei Raumtemperatur eine Reaktionszeit von höchstens 10, vorzugsweise etwa 5 Minuten benötigt,
- bei visueller Beurteilung semiquantitative Ergebnisse liefert,
- bei remissionsphotometrischer Auswertung eine quantitative Harnstoffbestimmung ermöglicht.

Diese Aufgabe wurde in der Weise gelöst, daß man eine Vorrichtung verwendet, die aus einem Handgriff (5), einer darauf befestigten Indikatorschicht für gasförmiges Ammoniak (1) und einer alkalischen Puffer enthaltenden Reaktionsschicht (3) besteht, wobei die Indikatorschicht fest mit dem Handgriff verbunden und darüber die Reaktionsschicht mittels eines Abstandshalters (2) angeordnet ist und Abstandshalter und Reaktionsschicht von der Indikatorschicht abgezogen werden können, wobei der Abstandshalter ein gewebtes oder gewirktes Netz oder eine Lochfolie mit einer Dicke von 10–200 µm und einer freien Lochfläche von mindestens 25% ist. Näher erläutert wird diese Vorrichtung in den nachstehenden Beispielen. Darüber hinaus lassen sich mit nach diesem Prinzip arbeitenden Teststreifen jedoch auch Ammoniak selbst wie Ammoniaksalze und unter Bildung von Ammoniak reagierende Substrate nachweisen und mengenmäßig bestimmten, indem statt der Urease mit dem jeweiligen Substrat unter Bildung von Ammoniak reagierende Reagenzien eingesetzt werden. Beispielhaft seien folgende Substrat/Reagenz-Kombinationen genannt:

Harnstoff/Urease
Creatinin/Creatinindeiminase
Aminosäure/Aminosäure-Dehydrogenase
Aminosäure/Aminosäure-Oxydase
Aminosäure/Aminosäure-Dehydratase
Aminosäure/Ammonia-Lyase
Amin/Amin-Oxydase
Diamin/Amin-Oxydase
Glucose/Phosphoramidathexosephosphortransferase und Phosphoramidat
ADP/Carbamatkinase und Carbamoulphosphat
Säureamid/Amidohydrolase
Nucleobase/Deaminase
Nucleosid/Deaminase
Nucleotid/Deaminase

Die Erfindung ist dadurch gekennzeichnet, daß eine Ammoniak bildende und gleichzeitig Ammoniak austreibende Reaktionsschicht in einem kleinen aber exakt reprodzierbaren Abstand über einer Indikatorschicht fixiert wird, ohne daß die freie Diffusion des Ammoniaks durch Filme, Schichten u. ä. behindert wird. Freie Diffusion und kleiner Abstand sind Voraussetzungen für eine kurze Reaktionszeit. Nur mit einem auch bei technischer Fertigung exakt reprodzierbaren Abstand beider Schichten kann ein solcher Testreifen quantitativen Anforderungen genügen.

Wie in Abb. 1 gezeigt, enthält der Teststreifen eine Indikatorschicht 1, die vorzugsweise aus einem saugfähigen Träger, z. B. Papier, einem porösen Kunststoff etc. besteht und ein Reagenzgemisch enthält, dessen remissionsphotometrische Eigenschaften sich bei Einwirkung von Ammoniakgas in Abhängigkeit von der Ammoniakmenge abgestuft ändern. Vorzugsweise enthält die Indikatorschicht

einen geeigneten pH-Indikator, bevorzugt aus der Reihe der Tetranitrodiphenylmethyl-pyridine und einen Puffer wie z. B. Citrat-, Tartrat- und Malonat-Puffer, aber auch polymere Puffer wie z. B. Polyacrylate oder Polymethacrylate bzw. deren Copolymere oder andere carboxylgruppenhaltige Polymere. Über die Indikatorschicht 1 wird ein Abstandshalter 2 gelegt, der sich sowohl durch eine konstante Dicke von ca. 10–200 µm als auch durch eine konstant offene Lochfläche von mindestens 25% auszeichnet. Er kann aus hydrophobierten gewebten oder gewirkten Netzen oder Lochfolien bestehen, die die vorstehenden Forderungen erfüllen. Vorzugsweise wird ein hydrophobiertes Gewebe verwendet. Solche Netze sind beispielsweise als Siebdruckgewebe oder Müllergaze bekannt.

Als Lochfolie kann eine Folie mit vielen relativ kleinen Löchern gewählt werden, die dann praktisch den gewebten Netzen entspricht, es kann jedoch auch eine Folie verwendet werden, die nur ein großes Loch genau über der Mitte des Testbezirks aufweist.

Über dem Abstandshalter 2 befindet sich die Reaktionsschicht 3, die vorzugsweise aus einem saugfähigen Träger, z. B. Papier, besteht. Sie enthält die Ammoniak bildenden Reagenzien, einen alkalischen Puffer, z. B. Ethylendiaminotetraacetat (EDTA), Tris(hydroxymethyl)aminomethan (TRIS) oder Phosphat, vom pH 7–9,5, vorzugsweise TRIS-Puffer pH 8,5 und Hilfsstoffe, z. B. Netzmittel und Stabilisatoren.

Die Reaktionsschicht wird vorzugsweise mit einem Netzwerk 4 (Gewebe, Gewirke o. ä.) gemäß DE-AS 2 118 455 festgehalten kann jedoch auch in üblicher Weise auf dem Abstandshalter festgeklebt oder angesiegelt sein.

Zur Substratbestimmung wird die Reaktionsschicht mit einem Tropfen Testflüssigkeit gefüllt. Um ein Entweichen des Ammoniaks nach oben zu verhindern, kann die Oberseite der Reaktionsschicht zweckmäßigerweise mit einer Abdeckschicht, beispielsweise einem Klebeetikett o. ä. verschlossen werden. Nach einer Reaktionszeit von 2–10 Minuten, normalerweise 5–7 Minuten, werden Reaktionsschicht und Abstandshalter abgezogen. Die Färbung der dadurch freigegebenen Indikatorschicht wird quantitativ mit einem Remissionsphotometer gemessen oder semiquantitativ durch visuellen Vergleich mit Vergleichsfarben bestimmt.

Die bevorzugt verwendeten Indikatoren aus der Reihe der Tetranitrodiphenylmethylpyridine sind neue Verbindungen die in der EP-A-0 005 518 beschrieben sind.

## Beispiel 1

### Quantitativer Nachweis von Harnstoff in Serum

#### a) Ureasepapier

Filterpapier wird mit einer Lösung folgender Zusammensetzung getränkt, getrocknet und in 6 mm breite Bänder zerschnitten.

| | |
|---|---|
| Urease (5 U/mg) | 6 g |
| Dithioerythrit | 0,1 g |
| 0,3 m TRIS.HCL-Puffer pH 8,5 | 100 ml |

#### b) Indikatorpapier

Filterpapier wird mit einer Lösung folgender Zusammensetzung getränkt, getrocknet und ebenfalls in 6 mm breite Streifen geschnitten.

| | |
|---|---|
| N-(Bis-(2,4-dinitrophenyl)-methyl)-4-t.butyl-pyridiniumchlorid | 0,39 g |
| Ethylenglykolmonomethylether | 42 ml |
| 0,25 m Na-Malonat-Puffer pH 2,8 | 48 ml |

#### c) Abstandshalter

Mit Silikonharz hydrophobiertes Siebdruckgewebe von ca. 100 µm Fadendicke und mit einer offenen Siebfläche von ca. 35% wird in 25–40 mm breite Bänder geschnitten.

d) Abdecknetz

Hydrophiles Nylonnetz von ca. 60 µm Dicke, Fadenstärke 40 µ, freie Lochfläche ca. 65% wird in 15 mm breite Bänder geschnitten.

e) Folie

Als Trägerfolie und Handgriff wird ein 60–100 mm breites, ca. 0,2–0,3 mm dickes Band aus schmelzkleberbeschichteter Polyesterfolie verwendet.

Herstellung der Teststreifen

Ureasepapier (3), Indikatorpapier (1) und Abstandshalter (2) werden zusammen mit einem das Ureasepapier abdeckenden Netz (4) wie in Abb. 1 gezeigt auf das Ende der mit Schmelzkleber beschichteten 6–10 cm breiten Folie (5) gesiegelt und das entstehende Band in 6 mm breite Streifen geschnitten, so daß 6 x 6 mm-Testbezirke an einem 6–10 cm langen Handgriff entstehen.

Ein solcher Streifen wird auf das Abdecknetz mit 10 µl Serum betropft und mit einem Klebeetikett verschlossen. Nach einer Reaktionszeit von 7 Minuten werden Ureasepapier und Abdecknetz zusammen mit der Trennschicht entfernt. Die Verfärbung der Indikatorschicht wird von oben mit einem Remissionsphotometer vermessen. In Abhängigkeit von der Harnstoffkonzentration werden folgende Meßwerte erhalten:

| mg Harnstoff/ 100 ml Serum | Meßsignal (Skalenteile) ± 1 s Mittelwert aus 10 Werten |
|---|---|
| 20 | 12,9 ± 0,75 |
| 40 | 27,5 ± 1,5 |
| 60 | 46,1 ± 1,9 |
| 80 | 61,5 ± 1,4 |
| 100 | 69,0 ± 0,4 |
| 150 | 77,2 ± 0,5 |
| 200 | 79,4 ± 0,5 |

Beispiel 2

Quantitativer Nachweis von Harnstoff in Blut

a) Ureasepapier

siehe Beispiel 1

b) Indikatorpapier

Filterpapier wird mit einer Lösung folgender Zusammensetzung getränkt und getrocknet

| | |
|---|---|
| N-(Bis-(2,4-dinitrophenyl)-methyl)-4-t.butyl-pyridiniumchlorid | 0,44 g |
| Ethylenglykolmonomethylether | 40 ml |
| Polyacrylat (Acrytex SL 865R der Firma Röhm) | 6 g |
| Wasser | 60 ml |

c) Abstandshalter

20 µm dicke Polypropylenfolie mit 35–45% offener Lochfläche

d) Abdecknetz

siehe Beispiel 1

e) Folie

siehe Beispiel 1

Verarbeitung und Harnstoffbestimmung werden wie in Beispiel 1 durchgeführt (jedoch unter Verwendung von AEDTA-Blutplasmen), wobei folgende Meßwerte erhalten werden:

| mg Harnstoff/ 100 ml Plasma | Meßsignal (Skalenteile) ± 1 s Mittelwert aus 10 Werten |
|---|---|
| 20 | 11,6 ± 0,6 |
| 40 | 23,0 ± 1,3 |
| 60 | 41,2 ± 1,9 |
| 80 | 56,7 ± 1,5 |
| 100 | 66,4 ± 0,5 |
| 150 | 75,3 ± 0,5 |
| 200 | 78,2 ± 0,4 |

Beispiel 3

Semiquantitativer Nachweis von Harnstoff in Blut

a) Ureasepapier

siehe Beispiel 1

b) Indikatorpapier

Filterpapier wird mit einer Lösung folgender Zusammensetzung getränkt und bei 70° C getrocknet

| | |
|---|---|
| Bromphenolblau | 0,1 g |
| Ethylenglykolmonomethylether | 9 ml |
| Weinsäure | 0,4 g |
| Wasser | 21 ml |

c) Abstandshalter

Mit Silikonharz hydrophob ausgerüstetes Polyamidvlies, Dicke ca. 80 µm

d) Abdecknetz

siehe Beispiel 1

Verarbeitung siehe Beispiel 1.

Zur Harnstoffbestimmung in Vollblut wird mit einem Tropfen Blut betropft. Nach einer Reaktionszeit von 7 Minuten entstehen je nach Harnstoffgehalt visuell gut unterscheidbare Reaktionsfarben:

| | |
|---|---|
| 20 mg Harnstoff/100 ml Blut | gelb |
| 40 mg Harnstoff/100 ml Blut | grünlich-gelb |
| 60 mg Harnstoff/100 ml Blut | gelbgrün |
| 80 mg Harnstoff/100 ml Blut | grün |
| 100 mg Harnstoff/100 ml Blut | blaugrün |
| 150 mg Harnstoff/100 ml Blut | grünlich-blau |
| 200 mg Harnstoff/100 ml Blut | blau |

Beispiel 4

Quantitativer Nachweis von Creatinin in Serum oder Blut

a) Creatinin-Deiminase-Papier

Filterpapier wird mit einer Lösung folgender Zusammensetzung getränkt, getrocknet und in 6 mm breite Bänder zerschnitten.

| | |
|---|---|
| Creatinin-Deiminase | 2000 U |
| Dithioerythrit | 0,1 g |
| 0,3 m TRIS · HCl Puffer pH 8,5 | 100 ml |

b) Indikatorschicht

Eine Masse folgender Zusammensetzung wird in einer Stärke von 0,1 mm auf eine Polycarbonat-Folie beschichtet, getrocknet und in 6 mm breite Bänder zerschnitten.

| | |
|---|---|
| N-(Bis-(2,4-dinitrophenyl)-methyl-4-t-butylpyridiniumchlorid | 0,15 g |
| Hydroxypropylcellulose Culminal PK 82® (Henkel) | 0,18 g |
| Wasser | 30 ml |
| 0,01 n HCl | 1,5 ml |

c) Abstandshalter

Mit Silikonharz hydrophobiertes Siebdruckgewebe von ca. 100 µm Fadenstärke und mit einer offenen Siebfläche von ca. 35% wird in 25—40 mm breite Bänder geschnitten.

d) Abdecknetz

Hydrophiles Nylonnetz von ca. 60 µm Dicke, Fadenstärke 40 µm, freie Lochfläche ca. 65% wird in 15 mm breite Bänder geschnitten.

e) Folie

Als Trägerfolie und Handgriff wird ein 60—100 mm breites, ca. 0,2—0,3 mm dickes Band aus schmelzkleberbeschichteter Polyesterfolie verwendet.

Herstellung der Teststreifen

Creatinin-Deiminase-Papier (3), Indikatorschicht (1) und Abstandshalter (2) werden zusammen mit einem das Creatinin-Deiminase-Papier abdeckenden Netz (4) wie in Abb. 1 gezeigt auf das Ende der mit Schmelzkleber beschichteten 6—10 cm breiten Folie (5) gesiegelt und das entstehende Band in 6 mm breite Streifen geschnitten, so daß 6 x 6 mm-Testbezirke an einem 6—10 cm langen Handgriff entstehen.

Ein solcher Streifen wird auf das Abdecknetz mit 10 µl Serum betropft und mit einem Klebeetikett

verschlossen. Nach einer Reaktionszeit von 7 Minuten werden Creatinin-Deiminase-Papier und Abdecknetz zusammen mit der Trennschicht entfernt. Die Verfärbung der Indikatorschicht wird von oben mit einem Remissionsphotometer vermessen. In Abhängigkeit von der Creatinin-Konzentration werden folgende Meßwerte erhalten:

| mg Creatinin/100 ml | Meßsignal (Skalenteile) |
|---|---|
| 0 | 28 |
| 0,5 | 37 |
| 1 | 47 |
| 2 | 60 |
| 3 | 68 |
| 4 | 73 |
| 5 | 76 |
| 6 | 78 |
| 7 | 80 |
| 8 | 82 |
| 9 | 83 |
| 10 | 84 |

Die auf diese Weise ermittelten Creatininkonzentrationen sind naturgemäß noch durch den im Serum ebenfalls vorhandenen Ammoniak verfälscht. Die wahren Creatininwerte erhält man, wenn man die gemäß Beispiel 5 erhaltenen Ammoniakkonzentrationen abzieht oder den Ammoniak, durch geeignete Maßnahmen (z. B. Ionenaustauscher) vor der Messung aus dem Serum entfernt.

Anmerkung

Die Verfälschung durch $NH_3$ fällt nur bei der Creatininbestimmung nicht jedoch bei der Harnstoffbestimmung gemäß Beispiel 1 und 2 ins Gewicht und zwar wegen des günstigeren Verhältnisses der vorkommenden Normalwerte im Serum:

| | |
|---|---|
| $NH_3$: | 0,016–0,038 mmol/l |
| Creatinin: | 0,044–0,097 mmol/l |
| Harnstoff: | 3,5–8,3 mmol/l |

Beispiel 5

Quantitativer Nachweis von Ammoniak in Serum oder Blut

a) Ammoniakaustreibendes Papier

Filterpapier wird mit einer wäßrigen Lösung von

0,3 m TRIS · HCL Puffer pH 9

getränkt, getrocknet und in 6 mm breite Bänder zerschnitten.

b) – e) Analog Beispiel 4

Herstellung und Anwendung des Teststreifens ebenfalls analog Beispiel 4.

In Abhängigkeit von der Ammoniakkonzentration werden folgende Meßwerte erhalten:

| $\mu g\ NH_3/100\ ml$ | Meßsignal (Skalenteile) |
|---|---|
| 0 | 27 |
| 50 | 41 |
| 100 | 50 |
| 150 | 54 |
| 200 | 59 |
| 300 | 65 |
| 600 | 75 |
| 1500 | 85 |

**Patentansprüche**

1. Schnelltest zur Bestimmung von Ammoniak oder unter Bildung von Ammoniak reagierenden Substraten einschließlich Vollblut bestehend aus einem Handgriff, einer darauf befestigten Indikatorschicht für gasförmiges Ammoniak und einer alkalischen Puffer enthaltenden Reaktionsschicht, die gegebenenfalls zusätzlich mit dem Substrat unter Bildung von Ammoniak reagierende Reagenzien enthält, dadurch gekennzeichnet, daß die Indikatorschicht fest mit dem Handgriff verbunden und darüber die Reaktionsschicht mittels eines Abstandshalters angeordnet ist und daß Abstandshalter und Reaktionsschicht von der Indikatorschicht abgezogen werden können, wobei der Abstandshalter ein gewebtes oder gewirktes Netz oder eine Lochfolie mit einer Dicke von 10 – 200 µm und einer freien Lochfläche von mindestens 25% ist.

2. Schnelltest nach Anspruch 1, dadurch gekennzeichnet, daß die Reaktionsschicht durch eine hydrophile, flüssigkeitsdurchlässige Abdeckschicht (4), vorzugsweise durch ein gewebtes oder gewirktes Netz auf dem Handgriff befestigt ist.

3. Schnelltest nach einem der Ansprüche 1 – 2, dadurch gekennzeichnet, daß das Substrat Harnstoff ist und das damit reagierende Reagenz Urease ist.

4. Schnelltest nach einem der Ansprüche 1 – 2, dadurch gekennzeichnet, daß das Substrat Creatinin ist und das damit reagierende Reagenz Creatinin-Deiminase ist.

5. Schnelltest gemäß einem der Ansprüche 1 bis 4, dadurch gekennzeichnet, daß eine Bis-(2,4-dinitrophenyl)-methyl-pyridinium-Verbindung der allgemeinen Formel I

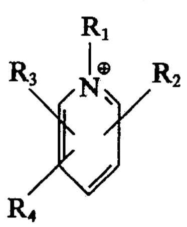 $X^{\ominus}$ (I)

wobei eine der Gruppen $R_1$ und $R_2$ eine Bis-(2,4-dinitrophenyl)-methylgruppe und die andere eine niedere Alkylgruppe, insbesondere Methyl- oder t.Butylgruppe oder im Falle von $R_2$ Wasserstoff bzw. eine Trifluormethylgruppe darstellt,
und $R_3$ und $R_4$ Wasserstoff oder zusammen mit den Kohlenstoffatomen, an die sie gebunden sind, einen Benzolring darstellen,
und $X^{\ominus}$ ein geeignetes Säureanion darstellt bzw. die Verbindung als Betain vorliegt
als Indikator enthalten ist.

6. Verfahren zum Nachweis von Ammoniak oder unter Bildung von Ammoniak reagierenden Substraten in wäßrigen Lösungen einschließlich Vollblut, dadurch gekennzeichnet, daß man die Reaktionsschicht einer Vorrichtung des Schnelltests nach den Ansprüchen 1 – 2, mit einem Tropfen

Ammoniak oder der unter Bildung von Ammoniak reagierenden Substrate tränkt, die Freisetzung von Ammoniak abwartet, die Indikatorschicht durch Abziehen der Reaktionsschicht und der Abstandsschicht freilegt und ihre Verfärbung visuell oder photometrisch bestimmt.

7. Verfahren zum Nachweis von Harnstoff in wäßrigen Lösungen, dadurch gekennzeichnet, daß man die Reaktionsschicht einer Vorrichtung des Schnelltests nach den Ansprüchen 1–2 mit einem Tropfen der harnstoffhaltigen Lösung tränkt, die Freisetzung von Ammoniak abwartet, die Indikatorschicht durch Abziehen der Reaktionsschicht und der Abstandsschicht freilegt und ihre Verfärbung visuell oder photometrisch bestimmt.

8. Verfahren zum Nachweis von Creatinin in wäßrigen Lösungen, dadurch gekennzeichnet, daß man die Reaktionsschicht einer Vorrichtung des Schnelltests nach den Ansprüchen 1–2 mit einem Tropfen der creatininhaltigen Lösung tränkt, die Freisetzung von Ammoniak abwartet, die Indikatorschicht durch Abziehen der Reaktionsschicht und der Abstandsschicht freilegt und ihre Verfärbung visuell oder photometrisch bestimmt.

## Claims

1. Rapid test for the determination of ammonia or of substrates reacting with the formation of ammonia, including whole blood, consisting of a handle, an indicator layer for gaseous ammonia fixed thereon and a reaction layer containing an alkaline buffer, which optionally additionally contains reagents reacting with the substrate with the formation of ammonia, characterized in that the indicator layer is securely connected to the handle and the reaction layer is arranged thereabove by means of a distance piece and in that the distance piece and the reaction layer can be pulled off the indicator layer, whereby the distance piece is a woven or knitted mesh or a perforated foil with a thickness of 10–200 µm. and a free hole surface of at least 25%.

2. Rapid test according to claim 1, characterised in that the reaction layer is fixed on to the handle by a hydrophilic, liquid-permeable covering layer (4), preferably by a woven or knitted mesh.

3. Rapid test according to one of claims 1–2, characterised in that the substrate is urea and the reagent reacting therewith is urease.

4. Rapid test according to one of claims 1–2, characterised in that the substrate is creatinine and the reagent reacting therewith is creatinine deiminase.

5. Rapid test according to one of claims 1 to 4, characterised in that , as indicator, it contains a bis-(2,3-dinitrophenyl)-methylpyridinium compound of the general formula I

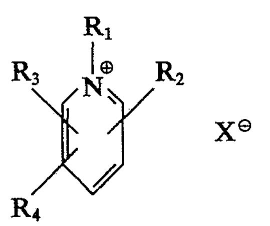

(I)

whereby one of the groups $R_1$ and $R_2$ represents a bis-(2,4-dinitrophenyl)-methyl group and the other a lower alkyl group, especially a methyl or t-butyl group, or, in the case of $R_2$, represents hydrogen or a trifluoromethyl group, and $R_3$ and $R_4$ represent hydrogen or, together with the carbon atoms to which they are attached, a benzene ring, and $X^{\ominus}$ represents a suitable acid anion or the compound is present as betaine.

6. Process for the detection of ammonia or of substrates reacting with the formation of ammonia in aqueous solutions, including whole blood, characterised in that one impregnates the reaction layer of a device of the rapid test according to claims 1–2 with a drop of ammonia or of the substrate reacting with the formation of ammonia, awaits the liberation of ammonia, exposes the indicator layer by pulling off the reaction layer and the distance piece and determines its coloration visually or photometrically.

7. Process for the detection of urea in aqueous solutions, characterised in that one impregnates the reaction layer of a device of the rapid test according to claims 1–2 with a drop of the urea-containing solution, awaits the liberation of the ammonia, exposes the indicator layer by pulling off the reaction layer and the distance piece and determines its coloration visually or photometrically.

8. Process for the detection of creatinine in aqueous solutions, characterised in that one impregnates the reaction layer of a device of the rapid test according to claims 1–2 with a drop of the creatinine-containing solution, awaits the liberation of ammonia, exposes the indicator layer by pulling off the reaction layer and distance piece and determines its coloration visually or photometrically.

**Revendications**

1. Dispositif de test rapide pour le dosage d'ammoniac ou de substrats réagissant en formant de l'ammoniac, y compris le sang complet, comprenant une poignée, et, fixée sur celle-ci, une couche d'indicateur pour ammoniac gazeux et une couche de réaction contenant un tampon alcalin, cette dernière renfermant éventuellement en outre des réactifs réagissant avec le substrat en formant de l'ammoniac, caractérisé en ce que la couche d'indicateur est reliée de façon fixe à la poignée et la couche d'indicateur à la précédente au moyen d'une couche d'écartement, et en ce que la couche d'écartement et la couche de réaction peuvent être détachées de la couche d'indicateur, la couche d'écartement étant formée par un réseau tissé ou à mailles, ou par une feuille perforée, d'une épaisseur comprise entre 10 et 200 µm et dont la surface perforée libre s'élève à au moins 25%.

2. Dispositif de test rapide selon la revendication 1, caractérisé en ce que la couche de réaction est fixée sur la poignée au moyen d'une couche de recouvrement (4) hydrophile, perméable aux liquides, de préférence au moyen d'un réseau tissé ou à mailles.

3. Dispositif de test rapide selon l'une des revendications 1 et 2, caractérisé en ce que le substrat est de l'urée et l'uréase est le réactif agissant sur l'urée.

4. Dispositif de test rapide selon l'une des revendications 1 et 2, caractérisé en ce que le substrat est la créatinine et la créatinine-déiminase est le réactif agissant sur la créatinine.

5. Dispositif de test rapide selon l'une des revendications 1 à 4, caractérisé en ce qu'il contient comme indicateur un dérivé de bis-(2,4-dinitrophényl) méthyl-pyridinium de formule générale I:

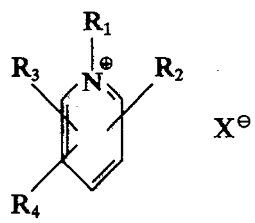

(I)

dans laquelle un des groupes $R_1$ et $R_2$ est un groupe bis-(2,4-dinitrophényl) méthyle et l'autre un groupe alkyle inférieur, en particulier un groupe méthyle ou t.butyle, ou, dans le cas où il s'agit de $R_2$, un atome d'hydrogène ou un groupe trifluorométhyle, et
$R_3$ et $R_4$ sont de l'hydrogène ou forment ensemble avec les atomes de carbone auxquels ils sont liés, un noyau benzénique,
et $X^{\ominus}$ est un anion d'acide approprié
ou bien le dérivé se présente sous forme d'une bétaïne.

6. Procédé de mise en évidence d'ammoniac ou de substrats réagissant en formant de l'ammoniac, y compris le sang complet, caractérisé en ce que l'on imprègne la couche de réaction d'un dispositif de test rapide selon l'une des revendications 1 et 2, d'une goutte d'ammoniac ou d'un substrat réagissant en formant de l'ammoniac, on attend le dégagement d'ammoniac, on met la couche d'indicateur à nu en détachant les couches de réaction et d'écartement et on détermine leur changement de couleur visuellement ou par voie photométrique.

7. Procédé de mise en évidence d'urée dans des solutions aqueuses, caractérisé en ce que l'on imprègne la couche de réaction d'undispositif de test rapide selon les revendications 1 et 2 d'une goutte de la solution contenant l'urée, on attend le dégagement d'ammoniac, on met la couche d'indicateur à nu en détachant les couches de réaction et d'écartement et on détermine leur changement de vouleur visuellement ou par voie photométrique.

8. Procédé de mise en évidence de créactinine dans des solutions aqueuses, caractérisé en ce que l'on imprègne la couche de réaction d'un dispositif de test rapide selon les revendications 1 et 2 d'une goutte de la solution contenant la créatinine, on attend le dégagement d'ammoniac, on met la couche d'indicateur à nu en détachant les couches de réaction et d'écartement et on détermine leur changement de couleur visuellement ou par voie photométrique.

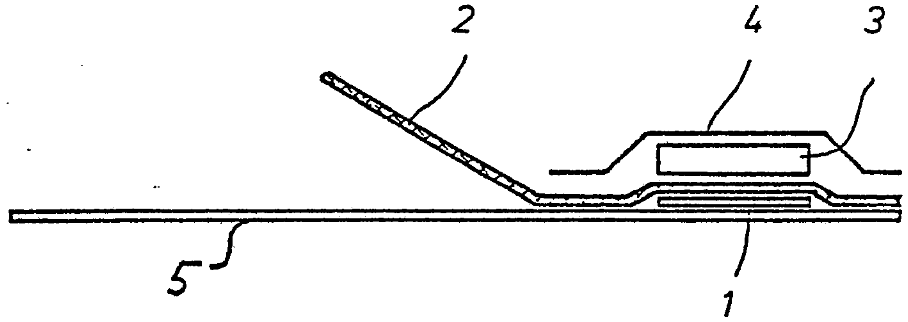


Abb. 1